(19) Europäisches Patentamt

European Patent Office

Office européen des brevets

(11) EP 0 801 643 B1

(12) EUROPÄISCHE PATENTSCHRIFT

(45) Veröffentlichungstag und Bekanntmachung des Hinweises auf die Patenterteilung:
**25.07.2001 Patentblatt 2001/30**

(21) Anmeldenummer: **95942729.5**

(22) Anmeldetag: **22.12.1995**

(51) Int Cl.[7]: **C07D 201/16**, C07D 201/08, C07B 63/02

(86) Internationale Anmeldenummer:
**PCT/EP95/05103**

(87) Internationale Veröffentlichungsnummer:
**WO 96/20923 (11.07.1996 Gazette 1996/31)**

(54) **VERFAHREN ZUR KONTINUIERLICHEN REINIGUNG VON AUS 6-AMINOCAPRONITRIL HERGESTELLTEM ROH-CAPROLACTAM**

PROCESS FOR CONTINUOUSLY CLEANING RAW CAPROLACTAM MADE FROM 6-AMINO CAPRONITRILE

PROCEDE DE PURIFICATION CONTINUE DE CAPROLACTAME BRUT PREPARE A PARTIR DE 6-AMINOCAPRONITRILE

(84) Benannte Vertragsstaaten:
**BE DE ES GB IT NL**

(30) Priorität: **03.01.1995 DE 19500041**

(43) Veröffentlichungstag der Anmeldung:
**22.10.1997 Patentblatt 1997/43**

(73) Patentinhaber: **BASF AKTIENGESELLSCHAFT**
**67056 Ludwigshafen (DE)**

(72) Erfinder:
 • **RITZ, Josef**
 **D-67069 Ludwigshafen (DE)**
 • **FISCHER, Rolf**
 **D-69121 Heidelberg (DE)**
 • **SCHNURR, Werner**
 **D-67273 Herxheim (DE)**
 • **ACHHAMMER, Günther**
 **D-68309 Mannheim (DE)**
 • **LUYKEN, Hermann**
 **D-67069 Ludwigshafen (DE)**
 • **FUCHS, Eberhard**
 **D-67227 Frankenthal (DE)**

(56) Entgegenhaltungen:
EP-A- 0 150 295      EP-A- 0 411 455
EP-A- 0 659 741      WO-A-95/14664
WO-A-95/14665        DD-A- 75 083
DE-A- 4 319 134      FR-A- 2 029 540
US-A- 2 301 964      US-A- 2 357 484
US-A- 3 145 198      US-A- 5 495 016
US-A- 5 496 941

 • CAN.J.CHEM.ENGINEER., Bd. 60, April 1982 Seiten 319-323, JODRA,L.G. ET AL 'Purification of epsilon-Caprolactam by Catalytic Hydrogenation '

**Beschreibung**

Die vorliegende Erfindung betrifft ein Verfahren zur kontinuierlichen Reinigung von Roh-Caprolactam durch Hydrierung, nachfolgende Behandlung im sauren Milieu und anschließende Destillation im alkalischen Milieu.

Es ist bekannt, Caprolactam durch Beckmann-Umlagerung von Cyclohexanonoxim mit Schwefelsäure oder Oleum herzustellen. Nach Neutralisation des Umlagerungsaustrags mit Ammoniak wird das freigesetzte Caprolactam durch Extraktion mit einem organischen Lösungsmittel von Ammonsulfat getrennt.

Je nach der Herstellmethode für die Cyclohexanonoxim-Ausgangsstoffe Cyclohexanon und Hydroxylammoniumsalz und der Oximierungs- und Umlagerungsmethode enthält das durch Beckmann-Umlagerung hergestellte Roh-Caprolactam Verunreinigungen, die sich nach Art und Menge unterscheiden. An die Reinheit des Caprolactams als Faserrohstoff werden hohe Anforderungen gestellt. Daher ist für jedes spezielle Verfahren zur Herstellung von Caprolactam aus Cyclohexanonoxim ein eigenes optimiertes Reinigungsverfahren notwendig.

So ist aus der DE-PS 1 253 716 ein Verfahren bekannt, bei dem man Caprolactam in Gegenwart von Hydrierkatalysatoren in Suspension oder in Rieselfahrweise unter Zusatz von Säuren wie Schwefelsäure hydriert. Nach einem ähnlichen, in der DE-PS 1 253 715 beschriebenen Verfahren setzt man bei der Hydrierung Alkali zu.

Nach einem anderen, in der DE-PS 1 004 616 beschriebenen Verfahren wird zu reinigendes Caprolactam zunächst mit Aktivkohle, dann mit Ionenaustauschern behandelt, anschließend in Gegenwart von Hydrierkatalysatoren in Suspension oder Rieselfahrweise hydriert und dann das hydrierte Caprolactam mit Ionenaustauschern behandelt.

Ferner ist aus der DD-PS 75 083 ein Verfahren zur Reinigung von Caprolactam bekannt, wobei man zunächst Caprolactam destilliert und dann Caprolactam gelöst in organischen Lösungsmitteln oder Wasser in Gegenwart eines fest angeordneten Skelett-Katalysators hydriert und anschließend das hydrierte Caprolactam mit Ionenaustauschern behandelt. In EP 411 455 wird gezeigt, daß man die für die Caprolactam-Qualität wichtigen Kennzahlen, Permanganatzahl und den Gehalt an flüchtigen Basen, gleichzeitig niedrig halten kann, wenn man das Rohcaprolactam kontinuierlich in Sumpffahrweise hydriert.

Neben der Beckmann-Umlagerung von Cyclohexanonoxim zu Caprolactam gibt es weitere zu Caprolactam führende Syntheserouten:

So ist bekannt, 6-Aminocapronitril mit Wasser in der Gas- oder Flüssigphase, in Gegenwart oder Abwesenheit von Katalysator, unter Freisetzung von Ammoniak zu Caprolactam umzusetzen:

Beim Erhitzen von 10 bis 25%igen wäßrigen Lösungen von 6-Aminocapronitril in der Flüssigphase auf 250 bis 290°C entsteht Caprolactam in bis zu 76%iger Ausbeute (US 2 301 964).

Weiterhin ist in der FR-A 2,029,540 die Cyclisierung von 25 bis 35%igen 6-Aminocapronitril-Lösungen bei 220°C in der Flüssigphase in Wasser unter Zusatz organischer Lösungsmittel in Gegenwart von z.B. Zink-, Kupfer-, Blei- und Quecksilberverbindungen beschrieben. Hierbei erzielt man Caprolactam-Ausbeuten von bis zu 83 %.

Die Cyclisierung von 6-Aminocapronitril läßt sich auch in der Gasphase durchführen. Ausgehend von 80%igen wäßrigen Lösungen erzielt man bei 305°C mit Aluminiumoxid als Katalysator Caprolactam-Ausbeuten von rund 92 % (US 2 357 484).

6-Aminocapronitril kann z.B. auch an Kupfer-Vanadin-Katalysatoren in der Gasphase bei 290°C in Gegenwart von Wasserstoff, Wasser und Ammoniak mit rund 77%iger Ausbeute zu Caprolactam umgesetzt werden (EP-A 150 295).

Das für die Cyclisierung benötigte 6-Aminocapronitril läßt sich z.B. durch partielle katalytische Hydrierung von Adipodinitril in Gegenwart von Ammoniak als Lösungsmittel herstellen: Hierbei kann man z.B. mit suspendierten Katalysatoren wie Rhodium auf Magnesiumoxid (US 4 601 859). Raney-Nickel (US 2 762 835, Freidlin et al., Russ. Chem, Rev. 33 (1964), WO 92/21650), Nickel auf Aluminiumoxid (US 2 208 598) oder Festbettkatalysatoren wie Kupfer/Cobalt/Zink- oder Eisen/Cobalt-Spinellen (DB 848 654), Cobalt auf Kieselgel (DB 954 416, US 2 257 814) oder Eisen (DE 42 35 466) arbeiten.

Nach WO 92/21650 werden z.B. in Gegenwart von Raney-Nickel Aminocapronitril-Ausbeuten von 60 % (Umsatz 70 %, Selektivität 86 %) und Hexamethylendiamin-Ausbeuten von 9 % erzielt. Bei einem Umsatz von 80 % beträgt die Aminocapronitril-Ausbeute 62 % (Selektivität 77 %).

Ein Reinigungsverfahren für Roh-Caprolactam, das aus 6-Aminocapronitril hergestellt wurde, ist bisher nicht bekannt. Da derartiges Roh-Caprolactam jedoch ein völlig anderes Nebenprodukt-Spektrum als durch Beckmann-Umlagerung hergestelltes Caprolactam besitzt, ist eine Übernahme der Reinigungsmethoden für Caprolactam, das durch Beckmann-Umlagerung erhalten wurde, nicht möglich.

So enthält aus Adipodinitril über 6-Aminocapronitril hergestelltes Roh-Caprolactam z.B. offenkettige und cyclische Nitrile, Amine und Imine als Nebenprodukte, die im Roh-Caprolactam aus der Beckmann-Umlagerung nicht anzutreffen sind.

Es war deshalb die technische Aufgabe gestellt, ein Reinigungsverfahren für aus 6-Aminocapronitril hergestelltes Caprolactam zur Verfügung zu stellen, das einen geringen Aufwand erfordert und auf sichere Weise zu spezifikationsgerechtem Caprolactam führt.

Demgemäß wurde ein Verfahren zur kontinuierlichen Reinigung von Roh-Caprolactam durch Hydrierung, nachfolgende Behandlung im sauren Milieu und anschließende Destillation im alkalischen Milieu gefunden, indem man

(a) 6-Aminocapronitril durch Umsetzung mit Wasser zu Roh-Caprolactam umsetzt,

(b) schwer- und leichtsiedende Anteile aus dem Roh-Caprolactam aus Schritt (a) abtrennt,

(c) das Roh-Caprolactam aus Schritt (b) bei einer Temperatur im Bereich von 50 bis 150°C und einem Druck im Bereich von 1,5 bis 250 bar in Gegenwart eines Hydrierungskatalysators und gewünschtenfalls eines Lösungsmittels mit Wasserstoff behandelt unter Erhalt einer Mischung A,

(d1) Mischung A in einem Lösungsmittel bei einer Temperatur im Bereich von 30 bis 80°C und einem Druck im Bereich von 1 bis 5 bar über einen Ionenaustauscher, enthaltend Säureendgruppen, leitet unter Erhalt einer Mischung B1, oder

(d2) Mischung A in Gegenwart von Schwefelsäure destilliert, wobei man vor Zugabe der Schwefelsäure gegebenenfalls vorhandenes Lösungsmittel entfernt, unter Erhalt einer Mischung B2, und

(e) Mischung B1 oder Mischung B2 in Gegenwart einer Base destilliert unter Erhalt von Rein-Caprolactam.

Erfindungsgemäß setzt man 6-Aminocapronitril in der Flüssig- oder Gasphase in Gegenwart von Wasser zu Caprolactam um. Verfahren zur Cyclisierung von 6-Aminocapronitril sind beispielsweise aus der US 2 245 129, US 2 301 964, EP-A 150,295 oder aus der FR-A 2 029 540 bekannt, so daß sich nähere Angaben hierzu erübrigen.

Das erfindungsgemäß als Ausgangsmaterial dienende 6-Aminocapronitril erhält man üblicherweise durch Hydrierung von Adipodinitril nach einem bekannten Verfahren, beispielsweise beschrieben in DE-A 836 938, DE-A 848 654 oder US 5 151 543.

In einer bevorzugten Ausführungsform setzt man 6-Aminocapronitril mit Wasser in flüssiger Phase unter Verwendung heterogener Katalysatoren um.

Die Umsetzung wird in flüssiger Phase bei Temperaturen von im allgemeinen 140 bis 320°C, vorzugsweise 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.

Pro mol 6-Aminocapronsäurenitril werden im allgemeinen mindestens 0,01 mol, vorzugsweise 0,1 bis 20 und insbesondere 1 bis 5 mol Wasser eingesetzt.

Vorteilhaft wird das 6-Aminocapronsäurenitril in Form einer 1 bis 50 gew.-%igen, insbesondere 5 bis 50 gew.-%igen, besonders vorzugsweise 5 bis 30 gew.-%igen Lösung in Wasser (wobei dann das Lösungsmittel gleichzeitig Reaktionspartner ist) oder in Wasser/ Lösungsmittel-Gemischen eingesetzt. Als Lösungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer Lösungsmittel Anwendung finden. Mischungen aus Wasser und Alkanolen im Gewichtsverhältnis Wasser/Alkanol 1-75/25-99, vorzugsweise 1-50/50-99 haben sich in einigen Fällen als besonders vorteilhaft herausgestellt.

Es ist prinzipiell genauso möglich, das 6-Aminocapronsäurenitril als Reaktand und gleichzeitig Lösungsmittel anzuwenden.

Als heterogene Katalysatoren können beispielsweise verwendet werden: Saure, basische oder amphotere Oxide der Elemente der zweiten, dritten oder vierten Hauptgruppe des Periodensystems, wie Calciumoxid, Magnesiumoxid, Boroxid, Aluminiumoxid, Zinnoxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titanoxid, amorph, als Anatas oder Rutil, Zirkonoxid, Zinkoxid, Manganoxid oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:

Vanadiniumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin sind Zeolithe, Phosphate und Heteropolysäuren, sowie saure und alkalische Ionenaustauscher wie beispielsweise Naphion® als geeignete Katalysatoren zu nennen.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Die Katalysatoren können je nach der Zusammensetzung des Katalysators als Vollkontakt oder Trägerkatalysator verwendet werden. So kann z.B. Titandioxid als Titandioxid-Strang oder als auf einen Träger in dünner Schicht aufgebrachtes Titandioxid eingesetzt werden. Zum Aufbringen von Titandioxid auf einen Träger wie Siliciumdioxid, Aluminiumoxid oder Zirkondioxid sind alle in der Literatur beschriebenen Methoden verwendbar. So kann eine dünne Titandioxid-Schicht durch Hydrolyse von Ti-Organylen wie Ti-Isopropylat oder Ti-Butylat, oder durch Hydrolyse von TiCl$_4$ oder anderen anorganischen Ti-haltigen Verbindungen aufgebracht werden. Auch Titandioxid-haltige Sole sind verwendbar.

Weitere geeignete Verbindungen sind Zirkonylchlorid, Aluminiumnitrat und Cernitrat.

Geeignete Träger sind Pulver, Stränge oder Tabletten der genannten Oxide selbst oder anderer stabiler Oxide wie Siliciumdioxid. Die verwendeten Träger können zur Verbesserung des Stofftransports makroporös ausgestaltet sein.

In einer weiteren bevorzugten Ausführungsform cyclisiert man 6-Aminocapronsäurenitril in Flüssigphase mit Wasser bei erhöhter Temperatur ohne Katalysator, indem man eine wäßrige Lösung von 6-Aminocapronsäurenitril in flüssiger Phase ohne Zusatz eines Katalysators in einem Reaktor erhitzt unter Erhalt einer Mischung I, bestehend aus im wesentlichen Wasser, Caprolactam und einer hochsiedenden Fraktion ("Hochsieder"). In dieser bevorzugten Ausführungsform setzt man Wasser bevorzugt im Überschuß ein, besonders bevorzugt verwendet man je mol 6-Aminocapronsäurenitril 10 bis 150, insbesondere 20 bis 100 mol Wasser unter Erhalt einer wäßrigen Lösung von 6-Aminocapronsäurenitril. In einer weiteren bevorzugten Ausführungsform verwendet man üblicherweise 5 bis 25 mol Wasser je mol 6-Aminocapronsäurenitril und kann die Lösung im allgemeinen durch Zusatz eines organischen Lösungsmittels auf 5 bis 25 Gew.-% 6-Aminocapronsäurenitril weiter verdünnen.

Als geeignete Lösungsmittel seien beispielsweise genannt:

C$_1$-C$_4$-Alkanole wie Methanol, Ethanol, n-, i-Propanol, Butanole, Glykole wie Ethylenglykol, Diethylenglykol, Triethylenglykol, Tetraethylenglykol, Ether wie Methyl-tert.-butylether, Diethylenglykoldiethylether, C$_6$-C$_{10}$-Alkane wie n-Hexan, n-Heptan, n-Octan, n-Nonan, n-Decan sowie Cyclohexan, Benzol, Toluol, Xylol, Lactame wie Pyrrolidon, Caprolactam oder N-C$_1$-C$_4$-Alkyl-Lactame wie N-Methylpyrrolidon, N-Methylcaprolactam oder N-Ethylcaprolactam.

In einer weiteren Ausführungsform kann man dem Reaktionsgemisch von 0 bis 5, bevorzugt von 0,1 bis 2 Gew.-% Ammoniak, Wasserstoff oder Stickstoff zusetzen.

Bevorzugt führt man die Reaktion bei einer Temperatur im Bereich von 200 bis 370, vorzugsweise von 220 bis 350°C, besonders bevorzugt von 240 bis 320°C durch.

Üblicherweise führt man die Reaktion unter Druck durch, wobei man den Druck in der Regel im Bereich von 0,1 bis 50, bevorzugt von 5 bis 25 MPa so wählt, daß das Reaktionsgemisch bevorzugt in flüssiger Phase vorliegt.

Die Reaktionsdauer hängt im wesentlichen von den gewählten Verfahrensparametern ab und liegt beim kontinuierlich durchgeführten Verfahren im allgemeinen im Bereich von 20 bis 180, bevorzugt von 20 bis 90 min. Bei kürzeren Reaktionszeiten sinkt in der Regel der Umsatz, bei längeren Reaktionszeiten bilden sich nach den bisherigen Beobachtungen störende Oligomere.

Die Cyclisierung führt man bevorzugt kontinuierlich, vorzugsweise in einem Rohrreaktor, in Rührkesseln oder Kombinationen davon durch.

Die Cyclisierung kann man auch diskontinuierlich durchführen. Die Reaktionsdauer liegt dann üblicherweise im Bereich von 30 bis 180 min.

Der Austrag ist in der Regel eine Mischung, bestehend im wesentlichen aus 50 bis 98, vorzugsweise von 80 bis 95 Gew.-% Wasser und von 2 bis 50, vorzugsweise von 5 bis 20 Gew.-% einer Mischung, bestehend im wesentlichen aus von 50 bis 90, vorzugsweise von 65 bis 85 Gew.-% Caprolactam und von 10 bis 50, vorzugsweise von 15 bis 35 Gew.-% einer hochsiedenden Fraktion ("Hochsieder").

In Schritt (b) des erfindungsgemäßen Verfahrens entfernt man schwer- und leichtsiedende Anteile aus dem in Schritt (a) erhaltenen Roh-Caprolactam, indem man Ammoniak, gegebenenfalls vorhandenes Lösungsmittel wie die zuvor genannten, insbesondere Alkohole, überschüssiges Wasser und unumgesetztes 6-Aminocapronsäurenitril sowie gegebenenfalls leichtsiedende Nebenprodukte durch Destillation, bevorzugt über Kopf, von Roh-Caprolactam und anschließend Roh-Caprolactam durch Destillation, bevorzugt über Kopf, von Hochsiedern wie Oligomeren der 6-Aminocapronsäure abtrennt. Nach bisherigen Beobachtungen ist es für den Erfolg der Erfindung nicht maßgeblich, ob die Leichtsieder vor den Hochsiedern oder umgekehrt oder gleichzeitig abgetrennt werden.

Erfindungsgemäß behandelt man das in Schritt (b) vorgereinigte Roh-Caprolactam mit Wasserstoff, wobei man das Roh-Caprolactam als Schmelze, bevorzugt in einem Lösungsmittel gelöst, einsetzt.

Als Lösungsmittel kommen bevorzugt solche in Frage, die sich unter den Bedingungen der Hydrierung und der Behandlung mit einem Ionenaustauscher inert verhalten. Insbesondere kommen in Betracht: $C_1$-$C_3$-Alkanole wie Methanol, Ethanol, n-Propanol, i-Propanol, vorzugsweise Ethanol, sowie, besonders bevorzugt, Wasser.

In einer bevorzugten Ausführungsform übernimmt man als Lösungsmittel dasjenige aus der Cyclisierung des 6-Aminocapronitrils, sofern dort ein Alkohol oder Wasser eingesetzt wurde.

Üblicherweise setzt man bei der Hydrierung 50 bis 95, vorzugsweise 70 bis 95 gew.-%ige Lösungen von Roh-Caprolactam, bezogen auf die Lösung, ein. Bei Übernahme des Lösungsmittels aus dem Cyclisierungsschritt kann es zur Erreichung der gewünschten Konzentration gegebenenfalls erforderlich sein, Lösungsmittel zuzufügen oder abzudestillieren.

Die Behandlung mit Wasserstoff führt man erfindungsgemäß bei einer Temperatur im Bereich von 50 bis 150, vorzugsweise von 60 bis 95, besonders bevorzugt von 70 bis 90°C in der Flüssigphase durch. Den Druck wählt man in Abhängigkeit von der Temperatur so, daß eine flüssige Phase aufrecht erhalten wird. Erfindungsgemäß liegt der Druck dabei in einem Bereich von 1,5 bis 250, vorzugsweise von 5 bis 100, besonders bevorzugt von 5 bis 20 bar.

Im allgemeinen setzt man Wasserstoff in Mengen im Bereich von 0,0001 bis 5,0, vorzugsweise von 0,001 bis 0,7, besonders vorzugsweise von 0,03 bis 0,3 mol je mol Caprolactam ein.

Die Verweilzeit beträgt in der Regel von 10 bis 300, bevorzugt von 15 bis 200 min.

Die Katalysatorbelastung wählt man üblicherweise im Bereich von 0,1 bis 15, bevorzugt von 1,5 bis 10 kg Caprolactam je Liter Katalysator und Stunde.

Die Hydrierung kann man sowohl in Suspension als auch in einem Festbett durchführen, wobei im letzten Fall bevorzugt eine Caprolactamlösung zusammen mit Wasserstoff von unten nach oben oder von oben nach unten über einen in einer rohrförmigen Zone fest angeordneten Katalysator geleitet wird.

Als Hydrierungskatalysatoren kann man nach bisherigen Beobachtungen solche bevorzugt einsetzen, die auf der Basis eines Metalles, ausgewählt aus der Gruppe, bestehend aus Eisen, Nickel, Cobalt, Ruthenium, Rhodium, Palladium, Osmium, Iridium und Platin, besonders bevorzugt Cobalt, Nickel und Palladium, ganz besonders bevorzugt Palladium, als Vollkatalysatoren oder Träger. katalysatoren, vorzugsweise in Form von Trägerkatalysatoren, erhältlich sind.

In einer bevorzugten Ausführungsform setzt man Palladiumträgerkatalysatoren ein, die einen Gehalt an Palladium im Bereich von 0,01 bis 10, vorzugsweise von 0,05 bis 5, besonders bevorzugt von 0,1 bis 2 Gew.-%, bezogen auf den Katalysator, aufweisen. Als Trägermaterial setzt man bevorzugt Aktivkohle, Aluminiumoxid, Zinkoxid, Siliciumdioxid, Titandioxid, Lanthanoxid oder Zirkondioxid, oder Mischungen davon, ein.

In einer weiteren bevorzugten Ausführungsform setzt man Nickel-trägerkatalysatoren ein, die einen Gehalt an Nickel im Bereich von 1 bis 80, vorzugsweise von 5 bis 50 Gew.-%, bezogen auf den Katalysator, aufweisen. Des weiteren kann der Nickelträgerkatalysator aktivierende Zusätze auf der Basis der Elemente Zirkonium, Mangan, Kupfer oder Chrom enthalten, wobei diese Zusätze in Mengen im Bereich von 0,1 bis 20, vorzugsweise von 1 bis 5 Gew.-%, bezogen auf die eingesetzte Menge an Nickel, im allgemeinen in oxidischer Form vorliegen.

Als Trägermaterialien verwendet man bevorzugt Aluminiumoxid, Kieselgel, Tonerden, Aktivkohle, Magnesiumsilikate, Aluminiumphosphat oder Borphosphat, besonders bevorzugt Magnesiumsilikate, Aluminiumphosphat, Borphosphat und Aluminiumoxid.

Angaben zur Herstellung derartiger Fällungs- oder Tränkkatalysatoren finden sich beispielsweise in Ullmanns Encyclopedia of Industrial Chemistry, Volume A5, Seiten 348-350, Fifth completely revised Edition.

In einer weiteren bevorzugten Ausführungsform verwendet man Trägerkatalysatoren, bei denen die katalytisch wirksamen Metalle an der Oberfläche angereichert sind. Solche Katalysatoren werden in der Regel nach an sich bekannten Methoden erhalten, indem man vorgeformte Träger aus den vorgenannten Stoffen in Form von Pellets, Kugeln oder Strängen mit einer wäßrigen Lösung der Metallsalze, beispielsweise der Nitrate, behandelt, diese dann trocknet, anschließend calciniert und dann mit Wasserstoff aktiviert.

In einer bevorzugten Ausführungsform ordnet man Palladium- oder Nickel-Trägerkatalysatoren in einer rohrförmigen Zone, beispielsweise mit einem Verhältnis von Länge zu Durchmesser im Bereich von 10:1 bis 50:1, fest an, beispielsweise als Schüttung, und leitet die (Roh-)Caprolactamlösung und Wasserstoff in Sumpf- oder Rieselfahrweise über das Bett des fest angeordneten Katalysators.

Durch die Behandlung mit Wasserstoff verbessern sich nach bisherigen Beobachtungen vor allem die UV-Zahl und die Permanganattitrationszahl (PTZ) des Roh-Caprolactams.

Nach dem Abkühlen und Entspannen erhält man eine Mischung A, die im wesentlichen aus Caprolactam und Lösungsmittel, sofern ein solches eingesetzt wird, besteht. Führt man die Hydrierung in einer Caprolactam-Schmelze durch, so wird in der Regel der Hydrieraustrag vor der Ionentauscher-Behandlung in einem der bei der Hydrierung zuvor genannten Lösungsmittel, bevorzugt Wasser, gelöst.

Erfindungsgemäß leitet man in Schritt (d1) Mischung A in einem Lösungsmittel bei einer Temperatur im Bereich von 30 bis 80, vorzugsweise von 50 bis 60°C, bei einem Druck im Bereich von 1 bis 5, vorzugsweise von 1 bis 2 bar, über einen Ionenaustauscher, der Säureendgruppen enthält unter Erhalt einer Mischung B1 als Austrag.

Als Ionenaustauscher setzt man vorzugsweise starksaure, d.h. Sulfonsäuregruppen enthaltende Ionenaustauscher in der H-Form ein. Geeignete Ionenaustauscher sind beispielsweise als Amberlite® , Dowex® oder Lewatit® im Handel (siehe z.B. Ullmanns Encyclopedia of Industrial Chemistry, Volume A14, Fifth completely revised Edition, Seite 451).

Die Belastung des Ionenaustauschers wählt man in der Regel im Bereich von 1 bis 15, vorzugsweise von 1 bis 10 kg Rohcaprolactam/l Ionenaustauscher und Stunde.

Durch die Behandlung mit dem Kationenaustauscher verbessert sich nach bisherigen Beobachtungen die UV-Zahl ein weiteres Mal.

Die Regenerierung der beladenen Ionenaustauscher ist üblicherweise durch Spülen mit wäßrigen Mineralsäuren wie Schwefelsäure oder Phosphorsäure möglich, wobei die auf dem Ionenaustauscher fixierten basischen Verbindungen im allgemeinen als wäßrige Lösungen der entsprechenden Salze ausgeschleust werden können.

Erfindungsgemäß kann man die Ionenaustauscher-Behandlung durch eine Destillation in Gegenwart von Schwefelsäure ersetzen (Schritt (d2)), wobei man gegebenenfalls vorhandenes Lösungsmittel vor Zugabe der Schwefelsäure entfernt.

In einer bevorzugten Ausführungsform entfernt man vorhandenes Lösungsmittel in einer Destillationskolonne mit zwei bis vier, besonders bevorzugt zwei bis drei theoretischen Böden bei einer Sumpftemperatur von maximal 145°C. Den Druck wählt man in Abhängigkeit von der gewählten Temperatur. Üblicherweise wählt man den Druck im Bereich von 35 bis 65 mbar, insbesondere 40 bis 60 mbar (gemessen am Destillationskopf), wenn die Sumpf temperatur 145°C beträgt.

Das so erhaltene bzw. bereits lösungsmittelfreies Roh-Caprolactam versetzt man erfindungsgemäß mit Schwefelsäure, im allgemeinen mit 0,1 bis 0,5, vorzugsweise 0,2 bis 0,3 Gew.-% Schwefelsäure (berechnet als 100 gew.-%ige Schwefelsäure), bezogen auf die Menge an Caprolactam.

Anschließend destilliert man unter Erhalt einer Mischung B2 und führt zweckmäßig den schwefelsäurehaltigen Destillationsrückstand einer Spaltschwefelsäure-Anlage zu. In einer bevorzugten Ausführungsform destilliert man in einer Destillationskolonne mit zwölf bis 18, vorzugsweise 14 bis 16 theoretischen Böden bei einem Kopfdruck im Bereich von drei bis sechs, vorzugsweise von drei bis vier mbar und einer Sumpftemperatur von maximal 145°C.

Die bei der Behandlung im sauren Milieu erhaltene Mischung B1 bzw. B2, entweder erhalten durch Behandlung mit einem Ionenaustauscher oder mit Schwefelsäure, destilliert man erfindungsgemäß (Schritt e) in Gegenwart einer Base. Als Base setzt man üblicherweise Alkalimetall- oder Erdalkalimetallverbindungen wie Hydroxide oder wasserlösliche Carbonate ein, wie Lithiumhydroxid, Natriumhydroxid, Kaliumhydroxid, Magnesiumhydroxid, Calciumhydroxid, Natriumcarbonat oder Mischungen davon, besonders bevorzugt Natriumhydroxid in Form von Natronlauge.

Die Menge an zugesetzter Base wählt man in der Regel im Bereich von 0,05 bis 0,9, vorzugsweise von 0,1 bis 0,8 mol-%, bezogen auf Caprolactam. In einer bevorzugten Ausführungsform setzt man 0,05 bis 0,25, vorzugsweise 0,1 bis 0,15 Gew.-% Natronlauge (berechnet als 100 gew.-%ig) zu.

Die Destillation kann man in an sich bekannter Weise durchführen, wobei man Lösungsmittel, Leicht- und Hochsieder von Caprolactam abtrennt.

In einer bevorzugten Ausführungsform destilliert man zuerst das Lösungsmittel, insbesondere Wasser, über Kopf aus der mit einer Base versetzten Mischung B1 oder B2 in der Regel in einer Destillationskolonne ab, wobei man eine Sumpftemperatur von maximal 160°C wählt und den Druck entsprechend einstellt. Bevorzugt arbeitet man bei einem Druck im Bereich von 35 bis 65, vorzugsweise von 40 bis 60 mbar (gemessen am Destillationskopf). Das Sumpfprodukt führt man zweckmäßig einer zweiten Destillationskolonne zu.

Das Sumpfprodukt der ersten Destillationskolonne destilliert man im allgemeinen in einer weiteren Destillationskolonne in der Regel bei einem Druck im Bereich von 4 bis 6, vorzugsweise von 4 mbar (gemessen am Kopf der Kolonne) und einer Sumpftemperatur von maximal 145°C. In dieser Destillationsstufe entfernt man üblicherweise leichtsiedende Anteile. Das Sumpfprodukt führt man bevorzugt einer dritten Destillationskolonne zu.

Das Sumpfprodukt der zweiten Kolonne führt man in der Regel einer weiteren Destillationskolonne zu, wobei man üblicherweise bei einem Druck im Bereich von 4 bis 6, vorzugsweise von 4 mbar, und bei einer Sumpftemperatur von maximal 145°C arbeitet. Das Kopfprodukt besteht nach bisherigen Beobachtungen aus spezifikationsgerechtem Rein-Caprolactam.

In einer weiteren bevorzugten Ausführungsform kann man das Sumpfprodukt der dritten Kolonne einem Fallfilmverdampfer zuführen, wobei weiteres Caprolactam abgetrennt werden kann, das man zweckmäßig in die erste Destillationskolonne rückführt.

Weiterhin bevorzugt ist die Variante, in der man als Base Natronlauge einsetzt. Hierbei kann man das natriumhaltige Sumpfprodukt der dritten Kolonne bzw. des Fallfilmverdampfers unter Gewinnung von Soda und Dampf einer Verbrennungsanlage zuführen.

Es ist auch möglich, die Trennoperationen der zweiten und dritten Kolonne in einer einzigen zusammenzufassen, indem man nur eine Destillationskolonne verwendet. Hierbei werden üblicherweise die Leichtsieder über Kopf, die Hochsieder über Sumpf und Caprolactam über einen Seitenabzug abgetrennt. Zweckmäßig führt man einen Teilstrom der leichtsiedenden Anteile ("Leichtsieder") nach Schritt (c) (Behandlung mit Wasserstoff) zurück.

Nach bisherigen Beobachtungen wird durch die destillative Aufarbeitung in Gegenwart einer Base die UV-Kennzahl weiter abgesenkt.

Durch die Folge der Reinigungsschritte Hydrierung, Behandlung im sauren Milieu und Destillation in Gegenwart einer Base, gelingt es nach dem erfindungsgemäßen Verfahren ein Reinlactam herzustellen, das in den Kennzahlen Permanganatabsorptionszahl (PAZ), Permanganattitrationszahl (PTZ), freie Basen, flüchtige Basen (FB) und UV-Kennzahl (UV) die Spezifikationswerte von Rein-Caprolactam, das durch Beckmann-Umlagerung erhalten wird, vollständig erfüllt. Der Gehalt an gaschromatographisch erfaßbaren Verunreinigungen liegt in der Regel im Bereich von 100 bis 150 ppm (bezogen auf Caprolactam). Da manche Verunreinigungen im Bereich von 10 ppm und weniger bereits die Einhaltung von Kennzahlen unmöglich machen können, und die Struktur zahlreicher Verunreinigungen in einem Mengenbereich von 10 ppm und weniger und deren chemisches Verhalten in Reinigungsschritten nicht bekannt sind, war der Erfolg des erfindungsgemäßen Verfahrens nicht vorherzusehen.

Beispiel

Die Reinigungsfolge wurde mit Rohcaprolactam durchgeführt, das durch Cyclisierung einer 10%igen ethanolischen 6-Aminocapronitril (ACN)-Lösung in Gegenwart von zwei Molen Wasser pro Mol ACN erhalten wurde:

In einen geheizten Rohrreaktor von 25 ml Inhalt (Durchmesser 6 mm; Länge 800 mm), der mit Titandioxid (Anatas) in Form von 1,5 mm Strängen gefüllt war, wurde bei 100 bar eine Lösung von 6-Aminocapronsäurenitril (ACN) in Wasser und Ethanol (10 Gew.-% ACN, 6,4 Gew.-% Wasser, Rest: Ethanol) geleitet, wobei die Reaktionstemperatur 240°C und die Verweilzeit 30 min betrugen. Der den Reaktor verlassende Produktstrom wurde gaschromatographisch und hochdruckflüssigchromatographisch (HPLC) analysiert: Umsatz: 100 %, Ausbeute: 88 %.

Der Reaktionsaustrag wurde durch fraktionierende Destillation von Hoch- und Leichtsiedern befreit. Das so erhaltene Rohcaprolactam besaß laut gaschromatographischer Analyse eine Reinheit von 99,5 %.

1000 g des Rohcaprolactams wurden in 250 g Wasser gelöst. Die wäßrige Lösung wurde in einem Autoklaven mit 3,5 g 5 gew.-%igem Palladium auf Aktivkohle als Träger versetzt und unter Rühren vier Stunden lang bei 80°C/5 bar hydriert.

Nach dem Abkühlen und Entspannen des Autoklaven wurde der Katalysator abfiltriert. Das Filtrat wurde bei 50°C und Normaldruck in Rieselfahrweise innerhalb von 0,6 Stunden über 1 l eines starksauren Ionentauschers (Amberlite® IR 120, H-Form) geleitet.

Der Ionentauscher-Austrag wurde mit 4 g 25 %iger wäßriger Natronlauge versetzt. In einer Destillationskolonne mit 2 theoretischen Böden wurde das Wasser bei einem Kopfdruck von 50 mbar und einer Sumpftemperatur von 135°C abdestilliert.

Aus dem Sumpfprodukt der ersten Kolonne wurden in einer zweiten Kolonne mit 15 theoretischen Böden die Leichtsieder bei einem Kopfdruck von 3,5 mbar und einer Sumpftemperatur von 140°C abdestilliert.

Das Sumpfprodukt der zweite Kolonne wurde in einer dritten Kolonne mit 15 theoretischen Böden destilliert. Bei einem Kopfdruck von 4 mbar und einer Sumpftemperatur von 145°C wurden insgesamt 990 g Caprolactam über Kopf destilliert (99 % bezogen auf eingesetztes Rohcaprolactam).

Das so erhaltene Reinlactam enthielt laut gaschromatographischer Analyse insgesamt nur 140 ppm Verunreinigungen, Verbindungen, welche die Polymerisation von Caprolactam zu Nylon 6 negativ beeinflussen könnten, wurden nicht gefunden. Die Kennzahlen des Reinlactams waren:

| PAZ | 1,5 |
|---|---|
| PTZ | 1,2 |
| freie Basen | < 0,05 meq/kg |
| flüchtige Basen | < 0,5 meq/kg |
| UV | 2,5. |

Damit erfüllte das aus 6-Aminocapronitril hergestellte Caprolactam die für "Beckmann-Caprolactam" geforderten Spezifikationswerte.

In Tabelle 1 ist die Verbesserung von UV-Kennzahl und Permanganattitrationszahl (PTZ) durch die einzelnen Reinigungsschritte demonstriert.

Tabelle 1

| Probe | UV-Kennzahl | Permanganattitrationszahl (PTZ) |
|---|---|---|
| Cyclisierungsaustrag | 110 | 400 |

Tabelle 1 (fortgesetzt)

| Probe | UV-Kennzahl | Permanganattitrationszahl (PTZ) |
|---|---|---|
| Hydrierungsaustrag | 40 | 390 |
| Ionentauscheraustrag | 15 | nicht gemessen |
| Reinlactam nach NaOH-Destillation | 2,5 | 1,2 |

Permanganattitrationszahl (PTZ)

Die Beständigkeit von Caprolactam gegenüber Kaliumpermanganat wurde titrimetrisch bestimmt. Die Permanganattitrationszahl (PTZ) entsprach dem Verbrauch an 0,1 N Kaliumpermanganat-Lösung in ml, berechnet auf 1 kg Caprolactam, der bei der Titration schwefelsaurer Lösung gefunden wurde.

Permanganatabsorptionszahl (PAZ)

Die Beständigkeit von Caprolactam gegenüber Kaliumpermanganat wurde photometrisch bestimmt (siehe auch ISO-Vorschrift 8660). Hierzu wurden gleiche Mengen einer 0,01 N Kaliumpermanganat-Lösung zu einer 3 % (m/m) wäßrigen Caprolactam-Lösung und zu einer Blindprobe (dest. Wasser) gegeben. Nach 10 Minuten wurden die Extinktionen E bei 420 nm sowohl der Caprolactamprobe als auch der Blindprobe verglichen. Die Permanganatabsorptionszahl berechnete sich aus der gemessenen Extinktion zu $(E-E_0)_{420} \cdot \frac{100}{3}$.

Flüchtige Basen (FB)

(Bestimmung in einer Apparatur nach Parnas; siehe auch ISO-Vorschrift 8661 "Caprolactam for industrial use - Determination of volatile bases content")

Bei einer Destillation im alkalischen Medium wurden die flüchtigen Basen aus der Probe freigesetzt (Kjeldahl-Apparatur), in einer 0,01 N Salzsäure aufgefangen und durch Titration mit 0,01 N Natronlauge bestimmt, die Einwaage betrug 20 + 0,1 g Caprolactam.

$$FB = \frac{(B-A) \times 0,01}{20} \times 1000 \text{ meq/kg}$$

A = Verbrauch an 0,01 N Natronlauge
B = Verbrauch an 0,01 N Natronlauge für eine Blindbestimmung

UV-Kennzahl (UV)

Die jeweiligen Extinktionen einer 50 % (m/m) wäßrigen Caprolactam-Lösung für 270, 280, 290, 300, 310, 320, 330, 340, 350 und 360 nm wurden in einer 10 cm-Küvette bestimmt. Die Summe der Extinktionswerte wurde mit 2 multipliziert und ergab die UV-Kennzahl bezogen auf 100 % Caprolactam.

Freie Basen

Zur Bestimmung der freien Basen wurden 150 ml destilliertes und mit Stickstoff begastes $CO_2$-freies Wasser mit 0,01 N Natronlauge auf genau pH 7,0 eingestellt und 50+/-0,1 g Caprolactam hinzugegeben. Anschließend wurde bei 25°C mit 0,01 N Salzsäure auf pH 7,0 titriert. Der Anteil an freier Base ließ sich dann nach untenstehender Formel berechnen, wobei A (ml) der Verbrauch an 0,01 N Salzsäure bedeutet:

Freie Basen = 0,01 * A * 1000/50 = 0,2 * A meq/kg

**Patentansprüche**

**1.** Verfahren zur kontinuierlichen Reinigung von Roh-Caprolactam durch Hydrierung, nachfolgende Behandlung im sauren Milieu und anschließende Destillation im alkalischen Milieu, dadurch gekennzeichnet, daß man

(a) 6-Aminocapronitril durch Umsetzung mit Wasser zu Roh-Caprolactam umsetzt,

(b) schwer- und leichtsiedende Anteile aus dem Roh-Caprolactam aus Schritt (a) abtrennt,

(c) das Roh-Caprolactam aus Schritt (b) bei einer Temperatur im Bereich von 50 bis 150°C und einem Druck im Bereich von 1,5 bis 250 bar in Gegenwart eines Hydrierungskatalysators und gewünschtenfalls eines Lösungsmittels mit Wasserstoff behandelt unter Erhalt einer Mischung A,

(d1) Mischung A in einem Lösungsmittel bei einer Temperatur im Bereich von 30 bis 80°C und einem Druck im Bereich von 1 bis 5 bar über einen Ionenaustauscher, enthaltend Säureendgruppen, leitet unter Erhalt einer Mischung B1, oder

(d2) Mischung A in Gegenwart von Schwefelsäure destilliert, wobei man vor Zugabe der Schwefelsäure gegebenenfalls vorhandenes Lösungsmittel entfernt, unter Erhalt einer Mischung B2, und

(e) Mischung B1 oder Mischung B2 in Gegenwart einer Base destilliert unter Erhalt von Rein-Caprolactam.

**2.** Verfahren nach Anspruch 1, dadurch gekennzeichnet, daß man als Lösungsmittel Wasser einsetzt.

**Claims**

**1.** A process for the continuous purification of crude caprolactam by hydrogenation, subsequent treatment in acidic medium and subsequent distillation in an alkaline medium, wherein

(a) 6-aminocapronitrile is converted into crude caprolactam by reaction with water,

(b) high boilers and low boilers are separated off from the crude caprolactam from step (a),

(c) the crude caprolactam from step (b) is treated with hydrogen at from 50 to 150°C and from 1.5 to 250 bar in the presence of a hydrogenation catalyst and, if desired, of a solvent to give a mixture A,

(d1) mixture A in a solvent is passed, at from 30 to 80°C and from 1 to 5 bar, over an ion exchanger containing terminal acid groups to give a mixture B1, or

(d2) mixture A is distilled in the presence of sulfuric acid, any solvent present being removed before the addition of the sulfuric acid, to give a mixture B2, and

(e) mixture B1 or mixture B2 is distilled in the presence of a base to give pure caprolactam.

**2.** A process as claimed in claim 1, wherein the solvent used is water.

**Revendications**

**1.** Procédé de purification continue de caprolactame brut par hydrogénation, traitement ultérieur en milieu acide et distillation ultérieure en milieu alcalin, caractérisé en ce que

(a) on transforme du 6-aminocapronitrile en caprolactame brut par réaction avec de l'eau,
(b) on isole des fractions à haut point d'ébullition et à bas point d'ébullition à partir du caprolactame brut de l'étape (a),
(c) en présence d'un catalyseur d'hydrogénation et éventuelement d'un solvant, on traite le caprolactame brut de l'étape (b) avec de l'hydrogène à une température de l'ordre de 50 à 150°C et à une pression de l'ordre de 1,5 à 250 bars, avec obtention d'un mélange A,
(d1) on fait passer le mélange A dans un solvant, à une température de l'ordre de 30 à 80°C et à une pression de l'ordre de 1 à 5 bars, sur un échangeur d'ions contenant des groupes terminaux acides, avec obtention d'un mélange B1, ou

(d2) on distille le mélange A en présence d'acide sulfurique, en éliminant, avant l'addition de l'acide sulfurique, du solvant éventuellement présent, avec obtention d'un mélange B2, et

(e) on distille le mélange B1 ou le mélange B2 en présence d'une base, avec obtention de caprolactame pur.

2. Procédé suivant la revendication 1, caractérisé en ce qu'on met en oeuvre de l'eau comme solvant.